(19) Europäisches Patentamt
European Patent Office
Office européen des brevets

(11) EP 0 811 622 B1

(12) **FASCICULE DE BREVET EUROPEEN**

(45) Date de publication et mention de la délivrance du brevet: **10.05.2000 Bulletin 2000/19**

(51) Int Cl.[7]: **C07D 413/14**, C07D 413/12, A61K 31/42

(21) Numéro de dépôt: **97401243.7**

(22) Date de dépôt: **04.06.1997**

(54) **Dérivés du 3-(pipérid-4-yl)-1,2-benzisoxazole et du 3-(pipérazin-4-yl)-1,2-benzisoxazole comme antipsychotiques**

3-(Piperid-4-yl)-1,2-benzisoxazol- und 3-(Piperazin-4-yl)-1,2-benzisoxazol-Derivate als Antipsychotika

3-(Piperid-4-yl)-1,2-benzisoxazole and 3-(piperazin-4-yl)-1,2-benzisoxazole derivatives as antipsychotics

(84) Etats contractants désignés:
**AT BE CH DE DK ES FI FR GB GR IE IT LI LU NL PT SE**

(30) Priorité: **04.06.1996 FR 9606866**

(43) Date de publication de la demande:
**10.12.1997 Bulletin 1997/50**

(73) Titulaire: **ADIR ET COMPAGNIE**
**92415 Courbevoie Cédex (FR)**

(72) Inventeurs:
- **Peglion, Jean-Louis**
  **78110 Le Vesinet (FR)**
- **Millan, Mark**
  **78230 Le Pecq (FR)**
- **Brocco, Mauricette**
  **75003 Paris (FR)**
- **Audinot, Valérie**
  **78300 Poissy (FR)**

(74) Mandataire: **Reverbori, Marcelle**
**ADIR**
**1, rue Carle Hébert**
**92415 Courbevoie Cédex (FR)**

(56) Documents cités:
**EP-A- 0 314 098**
**FR-A- 2 068 486**
**US-A- 4 352 811**

- **JOURNAL OF MEDICINAL CHEMISTRY, vol. 29, no. 3, 1 Mars 1986, pages 359-369, XP000561328 YEVICH J P ET AL: "SYNTHESIS AND BIOLOGICAL EVALUATION OF 1-(1,2-BENZISOTHIAZOL-3-YL)- AND (1,2-BENZISOXAZOL-3-YL)PIPERAZINE DERIVATIVES AS POTENTIAL ANTIPSYCHOTIC AGENTS"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 811 622 B1

Printed by Jouve, 75001 PARIS (FR)

## Description

La présente invention concerne de nouveaux composés du 1,2-benzisoxazole, leurs procédés de préparation et les compositions pharmaceutiques qui les contiennent.

De nombreux composés du 3-(pipérid-4-yl)1,2-benzisoxazole et du 3-(pipérazin-4-yl)1,2-benzisoxazole sont connus dans la littérature. Les brevets WO 9418197, US 4458076A et US 4352811A décrivent des composés qui se rapprochent le plus des composés de la présente demande. Ces composés sont doués de propriétés antipsychotiques ou analgésiques.

Les composés de la présente invention sont des composés du 3-(pipérid-4-yl)1,2-benzisoxazole et du 3-(pipérazin-4-yl)1,2-benzisoxazole qui se distinguent des composés déjà connus, par leur substituant en position 1 du noyau pipéridine ou pipérazine et par leurs propriétés pharmacologiques. En effet, différents essais pharmacologiques effectués, aussi bien in vitro qu' in vivo ont démontré que les composés de l'invention sont des antagonistes des récepteurs à la sérotonine $5HT_{2A}$, des récepteurs adrénergiques $\alpha_1$ et des récepteurs dopaminergiques. Ils possèdent une activité antipsychotique comparable à celle des produits de référence comme l'Halopéridol et la Rispéridone, mais surtout ils n'induisent pas d'effets secondaires, notamment d'effets extrapyramidaux. Il est connu que les antipsychotiques provoquent souvent des effets secondaires très importants ce qui limite leur utilisation. Les effets secondaires extrapyramidaux sont clairement reliés aux propriétés de blocage des récepteurs dopaminergiques $D_2$ par les molécules antipsychotiques utilisées en clinique. Or les composés de la présente invention sont des bloqueurs dopaminergiques $D_2$ beaucoup plus faibles que les produits connus. Par contre, leur affinité pour les récepteurs dopaminergiques $D_4$ est très élevée et nettement supérieure à leur affinité pour le récepteur $D_2$. Cette sélectivité explique donc l'absence d'effets secondaires de type extrapyramidaux démontrés avec les produits de la présente invention.
L'hyperactivité du système dopaminergique est impliquée, en plus de la schizophrénie, dans un nombre important de maladies de système nerveux central telle que les troubles anxio-dépressifs, les troubles de l'impulsion, l'agressivité. Les produits de l'invention sont donc plus particulièrement utilisés comme antipsychotiques, anxiolytiques et antiagressifs. Ils peuvent aussi être utilisés comme antalgiques.

La présente invention a plus particulièrement pour objet les composés du 1,2-benzisoxazole, de formule **I**

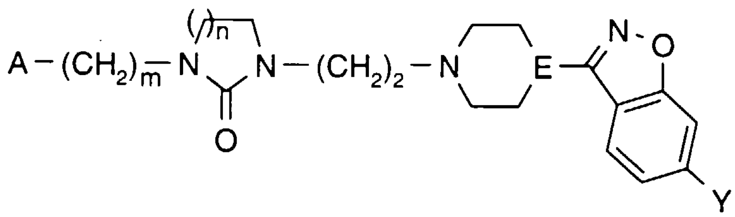

**(I)**

dans laquelle :

- A représente un radical alkyle linéaire ou ramifié de 1 à 10 atomes de carbone ou un radical phényle éventuellement substitué par un ou plusieurs atomes d'halogène ou groupements hydroxy ou alkoxy,
- m représente zéro ou 1,
- n représente 1 ou 2,
- E représente N ou CH, et
- Y représente un atome d'hydrogène ou d'halogène ou un radical alkoxy de 1 à 5 atomes de carbone ;

ainsi que leurs isomères optiques quand ils existent et leurs sels d'addition avec des acides organiques ou minéraux pharmaceutiquement acceptables.

La présente invention a également pour objet un procédé de préparation des composés de formule **I** caractérisé en ce que l'on fait réagir :
un composé de formule **II**

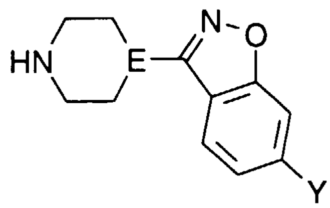

**(II)**

dans laquelle E et Y ont les significations précédemment définies,
<u>soit</u>
avec un composé de formule **III**

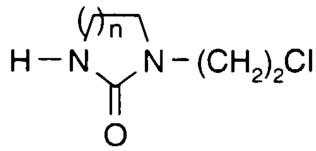

**(III)**

dans laquelle n a la signification précédemment définie, en présence de carbonate de métal alcalin tel que par exemple, le carbonate de potassium dans un solvant polaire comme, par exemple, la méthyléthylcétone ou la méthylisobutylcétone,
pour obtenir un composé de formule **IV**

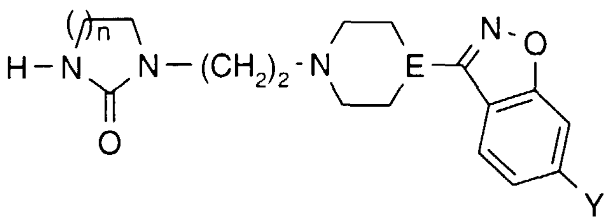

**(IV)**

dans laquelle n, E et Y ont les significations précédemment définies,
que l'on fait ensuite réagir avec un composé de formule **V**

$$A-(CH_2)_m-X \qquad \textbf{(V)}$$

dans laquelle A et m ont les significations précédemment définies et X est un atome d'halogène, en présence d'un hydrure de métal alcalin, tel que par exemple l'hydrure de sodium, dans un solvant polaire, comme par exemple le diméthylsulfoxyde,
pour obtenir les composés de formule I ;
<u>soit</u>
avec un composé de formule **VI**

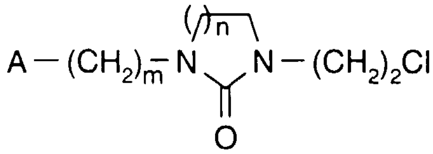

**(VI)**

dans laquelle A, m et n ont les significations précédemment définies, en présence de carbonate de métal alcalin, tel que le carbonate de potassium, dans un solvant polaire tel que l'acétone ou la méthylisobutylcétone pour former les

composés de formule **I**.

Les composés **III** et **VI** sont des produits connus ou préparés à partir de matières premières connues, selon des méthodes de synthèse classiques.

Les composés de la présente invention se différencient des composés de l'état antérieur de la technique non seulement par leur structure chimique mais aussi au niveau de leurs activités pharmacologiques et thérapeutiques. Ces activités ont été mises en évidence :

In vitro

par des études de binding sur les récepteurs clonés $D_2$ et $D_4$ humains et sur les récepteurs $5HT_{2A}$ et $\alpha_1$.

In vivo

- sur le modèle de verticalisation induite par l'apomorphine chez la souris,
- sur le modèle de conditionnement d'évitement actif chez le rat, et
- sur le modèle d'agressivité chez les souris isolées.

Enfin l'absence d'effets secondaires a pu être également vérifiée sur le modèle de catalepsie chez le rat.

Ces activités et l'absence d'effets secondaires gênants confèrent aux composés de la présente invention un intérêt tout particulier pour leur utilisation, à titre de médicaments, dans le traitement des troubles psychotiques, des troubles anxio-dépressifs et de l'agressivité.

La présente invention a également pour objet les compositions pharmaceutiques contenant comme principe actif un composé de formule I ou un de ses sels physiologiquement tolérable, mélangé ou associé à un ou plusieurs excipients pharmaceutiques appropriés.

Les compositions pharmaceutiques ainsi obtenues se présentent généralement sous forme dosée renfermant de 0,5 à 25 mg de principe actif. Elles peuvent, par exemple, revêtir la forme de comprimés, dragées, gélules, suppositoires, solutions injectables ou buvables et être administrées par la voie orale, rectale ou parentérale.

La posologie peut varier selon l''âge et le poids du patient, la voie d'administration, la nature de la maladie et les traitements associés et s'échelonne de 0,5 à 25 mg de principe actif, 1 à 3 fois par jour.

Les exemples suivants, donnés à titre non-limitatif, illustrent la présente invention. Les points de fusion ont été déterminés à la platine chauffante de Kofler sous microscope.

Exemple 1:

**1-{2-[4-(6-fluoro 1,2-benzisoxazol-3-yl)piperid-1-yl]ethyl}3-isopropyl imidazolidin-2-one**

Stade 1. 1-{2-[4-(6-fluoro 1,2-benzisoxazol-3-yl)piperid-1-yl]ethyl}imidazolidin-2-one On introduit dans un tricol 21,4 g (0,144 mole) de 1-(2-chloroéthyl) imidazolidin-2-one, 23,6 g (0,107 mole) de 4-(6-fluoro 1,2-benzisoxazol-3-yl) pipéridine, 92,9g g (0,67 mole) de carbonate de potassium, 2,6 g d'iodure de potassium et 524 ml de méthylisobutylcétone. On porte à reflux une nuit, puis concentre le mélange réactionnel et le reprend par un mélange eau/acétate d'éthyle. On décante, lave la phase organique plusieurs fois à l'eau, puis on l'extrait avec une solution normale d'acide chlorhydrique. La phase acide est ensuite basifiée à la soude et extraite au chlorure de méthylène. On sèche sur $MgSO_4$. Après évaporation, on recristallise le résidu dans 80 ml d'acétonitrile. On obtient alors 22 g d'un solide qui correspond au produit attendu. P.F. = 137-141° C (Rendement = 46 %).

Stade 2. Produit titre

5 g (0,015 mole) de produit obtenu au stade précédent sont dissous dans 16 ml de diméthylsulfoxyde. On rajoute par portions 1,2 (0,03 mole) d'hydrure de sodium à 60 %. On laisse en contact une demi-heure puis on coule 4,22 ml (0,045 mole) de 2-bromopropane. On laisse agiter la nuit à température ambiante. On reprend par $H_2O$, puis par du chlorure de méthylène. On décante, lave la phase organique avec de la saumure, la sèche sur $MgSO_4$ et la concentre sous vide. L'huile obtenue est chromatographiée sur silice (éluant : $CH_2Cl_2$ 95 / $CH_3OH$ 5) pour conduire au produit attendu. P.F. = 121-123° C (Rendement = 39 %).

Exemple 2:

**1-{2-[4-(6-fluoro 1,2-benzisoxazol-3-yl)pipérazin-1-yl]éthyl}3-isopropyl imidazolidin-2-one.**

Ce produit est préparé comme le composé de l'exemple 1 mais en utilisant au stade 1 la 4-(6-fluoro 1,2-benzisoxazol-3-yl)pipérazine à la place de la 4-(6-fluoro 1,2-benzisoxazol-3-yl)pipéridine. Le produit titre ainsi obtenu fond à 125-127° C.

Exemple 3:

**1-{2-[4-(6-fluoro 1,2-benzisoxazol-3-yl)pipérazin-1-yl]éthyl}3-éthyl imidazolidin-2-one.**

Ce produit est préparé comme le composé de l'exemple 1 mais en utilisant au stade 1 la 4-(6-fluoro 1,2-benzisoxazol-3-yl)pipérazine à la place de la 4-(6-fluoro 1,2-benzisoxazol-3-yl)pipéridine et au stade 2 le bromoéthane à la place du 2-bromopropane. Le chlorhydrate du produit titre ainsi obtenu fond à 201-204° C.

Exemple 4:

**1-{2- 1-{2-[4-(6-fluoro 1,2-benzisoxazol-3-yl)pipérid-1-yl]éthyl}3-isobutyl imidazolidin-2-one.**

Ce produit est préparé comme le composé de l'exemple 1 mais en utilisant au stade 2 le 1-bromo 2-méthyl propane à la place du 2-bromopropane.

Exemple 5 :

**1-{2-[4-(6-fluoro 1,2-benzisoxazol-3-yl)pipérid-1-yl]éthyl}3-phényl imidazolidin-2-one.**

Stade 1. 1,1-[di-(2-hydroxyéthyl)]3-phenyl urée.

Dans un tricol contenant 18,25 ml (0,168 mole) d'isocyanate de phényle dissous dans 37 ml de $CH_2Cl_2$ on coule, à une température de 10°C un mélange formé de 15,62 ml (0,136 mole) de diéthanolamine et de 163 ml de chlorure de méthylène. On laisse une heure à cette température, puis la nuit à température ambiante. On concentre sous vide pour obtenir 36,5 g de produit attendu. (Rendement = 100 %).

Stade 2. 1,1-[di-(2-chloroéthyl)]3-phényl urée.

On dissout 39 g (0,137 mole) de produit obtenu au stade précédent dans 100 ml de chlorure de méthylène. On coule, en maintenant la température à 0° C, 26,64 ml (0,365 mole) de chlorure de thionyle. On porte 4 h à reflux et abandonne une nuit à température ambiante. On concentre sous vide pour obtenir 43,7 g de produit attendu. (Rendement = 96 %).

Stade 3. 1-(2-chloroéthyl) 3-phényl imidazolidin-2-one.

43,7 g (0,167 mole) de produit obtenu au stade 2 sont portés à 120° C pendant 3 h, puis à 140°C pendant 6 h. A la fin du dégagement gazeux et après refroidissement on chromatographie l'huile obtenue sur silice (éluant : chlorure de méthylène 100 %). On obtient 23,2g d'un solide blanc qui correspond au produit attendu. P.F. = 92° C (Rendement = 61,7 %).

Stade 4. Produit titre

On procède comme décrit au stade 1 de l'exemple 1 mais en remplaçant la 1-(2-chloroéthyl)imidazolidin-2-one par la 1-(2-chloroéthyl)3-phényl imidazolidin-2-one. On obtient ainsi après purification par chromatographie sur silice (éluant : $CH_2Cl_2$ 80 / Cyclohexane 20) 3,8 g du produit attendu. P.F. = 158-160° C (Rendement = 56 %).

Exemple 6 :

**1-{2-[4-(6-fluoro 1,2-benzisoxazol-3-yl)pipérid-1-yl]éthyl}3-tertiobutyl imidazolidin-2-one.**

Ce produit est obtenu comme le composé de l'exemple 5, mais en remplaçant au stade 1 de cette synthèse l'isocyanate de phényle par l'isocyanate de tertiobutyle. Le chlorhydrate du produit titre attendu fond à 205-208° C.

Exemple 7 :

**3-benzyl 1-{2-[4-(6-fluoro 1,2-benzisoxazol-3-yl)pipérid-1-yl]éthyl} imidazolidin-2-one.**

Ce produit est obtenu comme le composé de l'exemple 5, mais en remplaçant au stade 1 de cette synthèse

l'isocyanate de phényle par l'isocyanate de benzyle. Le produit attendu fond à 126-130° C.

Exemple 8 :

**1-{2-[4-(6-fluorol 1,2-benzisoxazol-3-yl)pipérid-1-yl]éthyl}3-(2-éthoxyphényl) imidazolidin-2-one**

Ce produit est obtenu comme le composé de l'exemple 5, mais en remplaçant au stade 1 de cette synthèse l'isocyanate de phényle par l'isocyanate de 2-éthoxyphényle. Le chlorhydrate du produit titre fond à 214-218° C.

Exemple 9 :

**1-{2-[4-(6-fluoro 1,2-benzisoxazol-3-yl)pipérid-1-yl]éthyl}3-(2-chlorophényl) imidazolidin-2-one.**

Ce produit est obtenu comme le composé de l'exemple 5, mais en remplaçant au stade 1 de cette synthèse l'isocyanate de phényle par l'isocyanate de 2-chlorophényle. Le chlorhydrate du produit titre fond à 223-227° C.

Exemple 10 :

**1-{2-[4-(6-fluoro 1,2-benzisoxazol-3-yl)pipérid-1-yl]éthyl}3-(3-chlorophényl) imidazolidin-2-one.**

Ce produit est obtenu comme le composé de l'exemple 5, mais en remplaçant au stade 1 de cette synthèse l'isocyanate de phényle par l'isocyanate de 3-chlorophényle. Le chlorhydrate du produit titre fond à 223-227° C.

Exemple 11 :

**1-{2-[4-(6-fluoro 1,2-benzisoxazol-3-yl)pipérid-1-yl]éthyl} 3-(2,6-dichlorophényl) imidazolidin-2-one.**

Ce produit est obtenu comme le composé de l'exemple 5, mais en remplaçant au stade 1 de cette synthèse l'isocyanate de phényle par l'isocyanate de 2,6-dichlorophényle. Le chlorhydrate du produit titre fond à 237-241° C.

Exemple 12 :

**1-{2-[4-(6-fluoro 1,2-benzisoxazol-3-yl)pipérid-1-yl]éthyl}3-(4-méthoxyphényl) imidazolidin-2-one.**

Ce produit est obtenu comme le composé de l'exemple 5, mais en remplaçant au stade 1 de cette synthèse l'isocyanate de phényle par l'isocyanate de 4-méthoxyphényle. Le produit attendu fond à 154-157° C.

Exemple 13 :

**1-{2-[4-(6-fluoro 1,2-benzisoxazol-3-yl)pipérid-1-yl]éthyl}3-(2,3-dichlorophényl) imidazolidin-2-one.**

Ce produit est obtenu comme le composé de l'exemple 5, mais en remplaçant au stade 1 de cette synthèse l'isocyanate de phényle par l'isocyanate de 2,3-dichlorophényle. Le chlorhydrate du produit titre fond à 218-222° C.

Exemple 14 :

**1-{2-[4-(6-fluoro 1,2-benzisoxazol-3-yl)pipérid-1-yl]éthyl}3-(4-hydroxyphényl) imidazolidin-2-one.**

Ce produit est obtenu comme le composé de l'exemple 5, mais en remplaçant au stade 1 de cette synthèse l'isocyanate de phényle par l'isocyanate de 4-hydroxyphényle. Le produit attendu fond à 190-194° C.

Exemple 15:

**1-{2-[4-(6-fluoro 1,2-benzisoxazol-3-yl)pipérid-1-yl]éthyl} 3-phényl 3,4,5,6-tétrahydro-(2*H*)-pyrimidin-2-one.**

Stade 1. 1-phényl 3,4,5,6-tétrahydro-(2H)-pyrimidin-2-one

2,5 (16,5 mM)de $N^1$-phényl propane 1,3-diamine sont dissout dans 56,5 ml de tétrahydrofurane anhydre. On

coule sur cette solution un mélange de 3,8 g (23,3 mM) de carbonyldiimidazole dans 115 ml de tétrahydrofurane anhydre, puis abandonne une nuit à température ambiante. On concentre, reprend par de l'acétate d'éthyle, lave par HCl normal et sèche sur $MgSO_4$. On obtient 2g d'un solide blanc qui fond à 210-211°C et qui correspond au produit attendu.

Stade 2. 1-(2-chloroéthyl) 3-phényl 3,4,5,6-tétrahydro-(2*H*)-pyrimidin-2-one

1,76g (10mM) du produit obtenu au stade précédent sont dissout dans 15 ml de diméthylsulfoxyde anhydre. On ajoute par portions 0,81 g (20 mM) de NaH à 60 %. On laisse 2 h en contact puis on coule 1,7 ml (20 mM) de 1-bromo 2-chloroéthane. On abandonne un week-end à température ambiante. On ajoute 350 ml d'eau distillée puis on extrait au chlorure de méthylène. On décante, lave la phase organique, sèche sur $MgSO_4$. Après évaporation on obtient 1,8 g d'une huile qui correspond à la structure du produit attendu (Rendement = 76 %).

Stade 3. Produit titre

On procède comme décrit au stade 1 de l'exemple 1 mais en remplaçant la 1-(2-chloroéthyl) imidazolidin-2-one par la 1-(2-chloroéthyl) 3-phényl 3,4,5,6-tétrahydro-(2*H*)-pyrimidin-2-one obtenue ci-dessus. On obtient après purification par chromatographie sur silice (éluant : $CH_2Cl_2$ 80 / Cyclohexane 20) le produit attendu.

Exemple 16 :

Etude pharmacologique

In vitro

L'interaction des composés avec les récepteurs sérotoninergiques $5HT_{2A}$, adrénergiques $\alpha_1$ et dopaminergiques $D_2$ et $D_4$ a été déterminée en utilisant des études de fixation classique, d'après Millan *et al.* (J. Pharm. Exp. Ther., 275, 885-898, 1995).
Les conditions sont exposées ci-dessous :

| Récepteur | Espèce, tissu | $[^3H]$-ligand (nM) | NSB (10 µM) | Durée d'incubation, T° |
|---|---|---|---|---|
| $5HT_{2A}$ | rat, cortex frontal | Ketanserin (1,0) | Spiperone | 90 min, 25° C |
| $\alpha_1$ | rat, cortex frontal | Prazosin (0,2) | Phentolamine | 60 min, 25° C |
| $D_2$ | rat, striatum | Spiperone (0,2) | Raclopride | 30 min, 37° C |
| $D_4$ | humain, cellules CHO | Spiperone (0,2) | Haloperidol | 60 min, 27° C |

La séparation du radioligand lié aux récepteurs, du radioligand libre est effectuée par filtration sur des filtres GF/B prétraitées au polyéthylénimine 0.3 % grâce à un appareil de filtration de type Brandle Cell harvester. Du liquide scintillant est ajouté aux filtres dont la radioactivité est comptée à l'aide d'un compteur béta. L'$IC_{50}$ (concentration du composé qui inhibe 50 % de la liaison du radioligand) est déterminée par régression non linéaire. Le $K_i$ est ensuite calculé d'après l'équation de Cheng-Prussof : $K_i = IC_{50}/(1+L^*/K_d)$ où L* est la concentration de radioligand et $K_d$ la constante de dissociation du radioligand déterminée par expériences de saturation.
Les produits de l'invention ont des valeurs de Ki pour les récepteurs $5HT_{2A}$ et $\alpha_1$ inférieures à $10^{-8}$ M. En ce qui concerne la sélectivité $D_2$ par rapport à $D_4$ l'affinité pour le récepteur $D_4$ est généralement 10 fois supérieure à celle pour le récepteur $D_2$ ($\leq$ 5 nM pour $D_4$ vs $\geq$ 50 nM pour $D_2$)

In vivo

**1. Verticalisation induite par l'Apomorphine (0.75 mg/kg, s.c.) chez la souris.**

Ce test, décrit par Protais et al., Psychopharmacology, 50, 1-6 (1976), permet d'évaluer l'activité antagoniste dopaminergique de produits antipsychotiques éventuels. Une souris ayant reçu de l'apomorphine et placée dans une cage à barreaux verticaux, reste la plupart du temps, immobile en haut de la cage, accrochée par les quatre pattes aux barreaux. Ce comportement de verticalisation est bloqué si un produit antagoniste dopaminergique a été administré avant l'apomorphine.

***Test :*** dès l'administration sous-cutanée (s.c.) du produit ou du solvant (groupe contrôle) la souris est placée dans une cage grillagée cylindrique (14 cm diam. x 14 cm h), à barreaux verticaux. Trente minutes après, l'animal reçoit la dose d'apomorphine (0,75 mg/kg, s.c.). L'observation des animaux se fait à 10 et 20 minutes après l'injection d'apomorphine, avec attribution d'un *score 0* (quatre pattes au sol), *score 1* (souris redressée, deux pattes avant sur les barreaux) et *score 2* (souris accrochée par les quatre pattes sur les barreaux) à chaque temps de mesure. Le score de verticalisation utilisé pour les résultats est compris entre 0 et 4 (somme des 2 mesures). Chaque groupe expérimental comporte au moins 5 animaux.

***Analyse statistique :*** L'effet du produit sur la verticalisation est évalué en comparant les scores obtenus dans chaque groupe ayant reçu une dose de produit, à ceux obtenus dans le groupe contrôle (solvant), par un test U de Mann et Whitney, avec une probabilité $p < 0,05$.
La Dose Inhibitrice $ID_{50}$ est la dose de produit qui diminue de moitié la moyenne des scores de verticalisation par rapport à celle du groupe contrôle.

***Résultats :*** à titre d'exemple, le produit de l'exemple 5 à une $ID_{50}$ dans ce test de 0,22 mg/kg.

**2. Conditionnement d'évitement actif chez le rat**

Ce test est classiquement utilisé pour caractériser des produits antipsychotiques cf P.A. Janssen, C.J.E. Niemegeers, Arzneim-Forsch., 1037-1043 (1961).
Il s'effectue dans une cage à deux compartiments avec plancher électrifié, sur des rats qui ont été conditionnés de la façon suivante : dès la présentation d'un signal lumineux, l'animal doit passer du compartiment où il se trouve, dans l'autre compartiment, afin de ne pas recevoir de choc électrique. Cette réponse de l'animal au signal lumineux est une réponse conditionnée d'Evitement. Si l'animal n'effectue pas cette réponse, à la fin du signal lumineux, il reçoit un choc électrique jusqu'à ce qu'il aille dans l'autre compartiment. La réponse au choc électrique est une réponse inconditionnée d'Echappement.
Classiquement, les produits antipsychotiques inhibent la réponse conditionnée d'Evitement, à des doses inférieures à celles qui inhibent la réponse non conditionnée d'Echappement. Ceci les différencie d'autres classes de produits (en particulier, barbituriques, benzodiazépines), qui inhibent indifféremment les deux réponses.

***Matériel :*** Il consiste en une cage divisée en 2 compartiments par une cloison centrale; une ouverture dans cette cloison permet le passage de l'animal d'un compartiment à l'autre (modèle LE 916, LETICA). Le plancher de chaque compartiment est une grille électrifiée. Le fonctionnement de la cage (signal lumineux, passage de courant électrique dans la grille) et l'enregistrement des déplacements de l'animal d'un compartiment à l'autre, sont effectués par ordinateur (COMPAQ 386S) au moyen du logiciel SHUTTLE 8 (LETICA).

***Test :*** les animaux sont leur propres contrôles. Chaque session journalière comporte 10 essais séparés les uns des autres par un intervalle de 30 secondes. Un essai consiste en la présentation du signal lumineux (10 secondes), suivi ou non du choc électrique (0,460 mA, durée maximum 5 secondes) selon la réponse de l'animal au signal lumineux. Les effets d'un produit sur les réponses d'évitement sont évalués au cours d'une Session Test, qui a lieu le lendemain d'une Session Contrôle au cours de laquelle les animaux ont reçu le solvant. Le produit ou le solvant est administré à l'animal, 30 minutes avant le début de la Session. Le paramètre utilisé est le nombre de réponses d'évitement.

***Analyse statistique :*** Pour chaque dose de produit, le nombre de réponses d'évitement obtenu lors de la Session Test est comparé à celui obtenu chez les mêmes animaux lors de la Session Contrôle, en appliquant un Test de Wilcoxon, avec une probabilité $p < 0.05$. La Dose Inhibitrice moyenne $ID_{50}$ est celle qui réduit de 50 % le nombre d'évitements conditionnés par rapport à la valeur contrôle.

***Résultats :*** à titre d'exemple, le produit de l'exemple 5 a une $ID_{50}$ dans ce test de 0,88 mg/kg par voie sous-cutanée.

**3. Test d'agressivité chez des souris isolées**

Ce test permet d'évaluer l'activité anti-agressive intraspécifique d'un produit chez des souris qui ont été maintenues en isolement pendant plusieurs mois.

***Animaux*** *:* Le test utilise des souris mâles CD (Charles River) de poids 22 à 25 g à leur arrivée dans l'animalerie. Dès leur arrivée, les animaux sont isolés dans des cages individuelles en polycarbonate opaque noir (23 x 14 x 13 cm) avec un couvercle grillagé et stabulé de façon chronique (6 mois environ) dans la pièce d'expérimentation.

***Sélection des couples de souris :*** La sélection des couples de souris agressives qui seront utilisés de façon chronique dans l'étude, commence après un mois d'isolement des animaux. Une ou deux fois par semaine, on place dans la cage d'une souris (résidente), une souris d'une autre cage (intruse) et l'on observe si les deux animaux s'attaquent (reniflements, poursuites, mordillements, morsures) pendant cet essai. A la fin de l'essai (durée maximale de 10 min), chaque souris est isolée à nouveau dans sa cage respective. Si il y a eu des attaques, le même couple sera

testé à nouveau lors du prochain essai ; si il n'y a pas eu d'attaques, chaque souris de ce couple sera mise en présence d'une autre souris lors de l'essai suivant. On sélectionne ainsi, au cours d'essais successifs, à raison de 1 ou 2 essais par semaines, les couples définitifs de souris qui seront utilisés pour les expériences. La sélection des couples est basée sur la stabilité de la combativité des animaux d'un essai à l'autre, la faible latence de la première attaque et la fréquence et durée des attaques. Chez les couples ainsi sélectionnés, ces paramètres sont vérifiés chaque semaine au cours d'un essai rapide, sans traitement, deux jours avant le jour Test.

***Test :*** Le test a lieu une fois par semaine. Trente minutes avant leur mise en présence, les deux souris du couple reçoivent chacune le même traitement (produit ou solvant) et restent isolées dans leur cage respective. A T0 minute, la souris intruse est introduite dans la cage de la souris résidente pour une durée de trois minutes. On note la latence (en secondes) de la première attaque, le nombre et la durée totale (en secondes) des attaques. On note aussi une inversion éventuelle de dominance d'une souris par rapport à l'autre (en général, la souris résidente est la souris dominante).

A la fin du test, la souris intruse retourne dans sa cage ; les animaux restent en isolement jusqu'au prochain essai rapide et test, la semaine suivante.

***Analyse statistique :*** Les effets d'un produit sur l'agressivité sont évalués en comparant le nombre et la durée des attaques des couples ayant reçu le produit (groupes traités) à ceux obtenus chez les couples ayant reçu le solvant (groupe contrôle), en utilisant une analyse de variance (ANOVA) suivie d'un test de Dunnett, avec une probabilité $p < 0.05$.

La Dose Inhibitrice $ID_{50}$ du nombre ou de la durée des attaques est la dose de produit qui réduit de moitié la moyenne de chacune de ces valeurs, par rapport à celle obtenue respectivement dans le groupe contrôle.

***Résultats*** *:* à titre d'exemple, le produit de l'exemple 5 à une $ID_{50}$ dans ce test de 0,18 mg/kg par voie sous-cutanée.

**4. Induction de Catalepsies chez le Rat**

L'administration prolongée de neuroleptiques ou antipsychotiques "typiques" (Haloperidol, Chlorpromazine) chez des patients schizophrènes entraîne souvent l'apparition de signes extrapyramidaux (EPS) indésirables de type Parkinson, en particulier un phénomène d'immobilité cf. Davis *et al.,* "Neuroleptics : Neurochemical, Behavioral, and Clinical Perspectives", Eds. Coyle, J.T. and Enna, S.J. Raven Press, New York (1983). Par contre, les antipsychotiques "atypiques" (Clozapine) provoquent peu de signes extrapyramidaux.

Chez l'animal, l'administration aiguë d'antipsychotiques "typiques" induit une catalepsie, c'est-à-dire le maintien de l'animal dans une posture, souvent anormale, qui lui a été imposée par l'expérimentateur cf. Waldmeier P.C. et *al ;* Eur. J. of Pharmacology, 55, 363-373 (1979). L'évaluation des propriétés cataleptogènes d'un produit chez le rat permet donc de savoir si ce produit administré chez l'homme, risque de provoquer ou non, un syndrôme de type extrapyramidal.

***Test :*** les animaux sont placés en cage individuelle et mis à jeûn la veille du test, avec boisson à volonté. Le test de catalepsie consiste à placer chaque patte arrière de l'animal sur la patte avant du même côté et à mesurer le temps (secondes) pendant lequel l'animal garde cette position "pattes croisées" (maximum 30 secondes). Chaque animal est soumis à trois essais successifs (un toutes les deux minutes), l'animal étant retiré de sa cage et placé sur le plan de travail. Ces essais ont lieu 1 heure après injection sous-cutanée ou administration orale du produit ou de son solvant. La valeur moyenne des trois essais représente la durée de catalepsie (en secondes) pour chaque animal. Il y a cinq à six rats par groupe expérimental.

***Analyse statistique*** *:* L'effet du produit sur la durée de catalepsie est évalué par une ANOVA, suivie d'un test de Dunnett, avec une probabilité $p < 0.05$.

La Dose Active moyenne $AD_{50}$ d'induction de catalepsies est celle qui provoque une catalepsie d'une durée de 50 % par rapport à la valeur maximale de 30 secondes (corrigée de la valeur du groupe contrôle solvant).

***Résultats :*** à titre d'exemple, le produit de l'exemple 5 à une $AD_{50}$ dans ce test de 34 mg/kg, ce qui le compare très favorablement aux composés de référence comme l'Halopéridol ou la Rispéridone qui, dans ce test, ont des $AD_{50}$ respectives de 0,15 mg/kg et de 1,2 mg/kg par voie sous-cutanée.

**Revendications**

1. Les composés du 1,2-benzisoxazole, de formule I

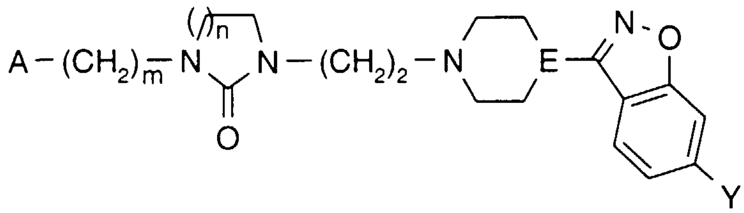

(I)

dans laquelle :

A est alkyle linéaire ou ramifié de 1 à 10 atomes de carbone ou phényle éventuellement substitué par un ou plusieurs halogène, hydroxy ou alkoxy,

m représente zéro ou 1,

n représente 1 ou 2,

E représente N ou CH, et

Y représente hydrogène, halogène ou ($C_1$-$C_5$)alkoxy ;

ainsi que leurs isomères optiques quand ils existent et leurs sels d'addition avec des acides pharmaceutiquement acceptables.

**2.** Un dérivé de la revendication 1 qui est le 1-{2-[4-(6-fluoro 1,2-benzisoxazol-3-yl)pipérazin-1-yl]éthyl}3-isopropyl imidazolidin-2-one.

**3.** Un dérivé de la revendication 1 qui est le 1-{2-[4-(6-fluoro 1,2-benzisoxazol-3-yl)pipérazin-1-yl]éthyl}3-éthyl imidazolidin-2-one et son chlorhydrate.

**4.** Un dérivé de la revendication 1 qui est le 1-{2-[4-(6-fluoro 1,2-benzisoxazol-3-yl)pipérid-1-yl]éthyl}3-phényl imidazolidin-2-one.

**5.** Le procédé de préparation des composés de la revendication 1 caractérisé en ce que l'on fait réagir :
un composé de formule **II**

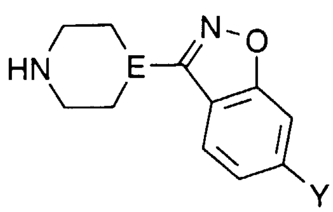

(II)

dans laquelle E et Y ont les significations définies dans la revendication 1, soit
avec un composé de formule **III**

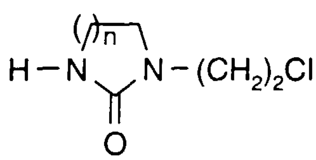

(III)

dans laquelle n a la signification précédemment définie,
en présence d'un carbonate de métal alcalin dans un solvant polaire,
pour obtenir un composé de formule **IV**

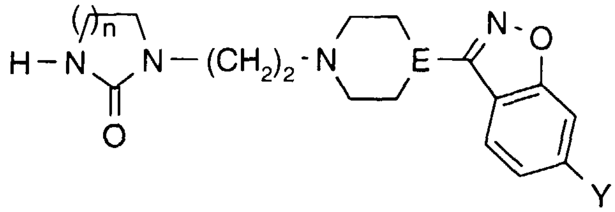

**(IV)**

dans laquelle n, E et Y ont les significations définies dans la revendication 1, que l'on fait ensuite réagir avec un composé de formule **V**

$$A-(CH_2)_m-X \qquad \textbf{(V)}$$

dans laquelle A et m ont les significations définies dans la revendication 1 et X est un atome d'halogène, en présence d'un hydrure de métal alcalin, dans un solvant polaire, pour obtenir les composés de formule I ;
soit
avec un composé de formule **VI**

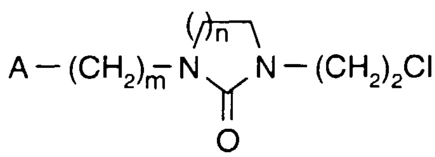

**(VI)**

dans laquelle A, m et n ont les significations définies dans la revendication 1, en présence d'un carbonate de métal alcalin, dans un solvant polaire, pour former les composés de formule **I**.

**6.** Les compositions pharmaceutiques utilisables dans le traitement des troubles psychotiques des troubles anxio-depressifs et de l'agressivité, contenant comme principe actif un composé selon une des revendications 1 à 4, avec un ou plusieurs excipients pharmaceutiques appropriés.

**Patentansprüche**

**1.** 1,2-Benzisoxazol-Verbindungen der Formel **I**

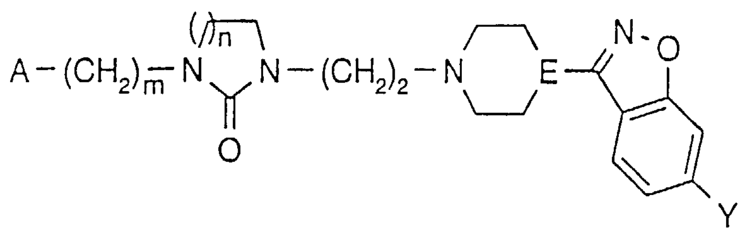

**(I)**

in der

A eine geradkettige oder verzweigte Alkylgruppe mit 1 bis 10 Kohlenstoffatomen oder eine Phenylgruppe, die gegebenenfalls durch ein oder mehrere Halogenatome, Hydroxygruppen oder Alkoxygruppen substituiert ist,
m Null oder 1,
n 1 oder 2,
E N oder CH und
Y Wasserstoff, Halogen oder ($C_1$-$C_5$)-Alkoxy bedeuten,

sowie deren optische Isomere wenn sie existieren und deren Additionssalze mit pharmazeutisch annehmbaren Säuren.

2. Derivat nach Anspruch 1, nämlich 1-{2-[4-(6-Fluor-1,2-benzisoxazol-3-yl)-piperazin-1-yl]-ethyl}-3-isopropyl-imidazolidin-2-on.

3. Derivat nach Anspruch 1, nämlich 1-{2-[4-(6-Fluor-1,2-benzisoxazol-3-yl)-piperazin-1-yl]-ethyl}-3-ethyl-imidazolidin-2-on und dessen Hydrochlorid.

4. Derivat nach Anspruch 1, nämlich 1-{2-[4-(6-Fluor-1,2-benzisoxazol-3-yl)-piperid-1-yl]-ethyl}-3-phenyl-imidazolidin-2-on.

5. Verfahren zur Herstellung der Verbindungen nach Anspruch 1, dadurch gekennzeichnet, daß man: eine Verbindung der Formel **II**

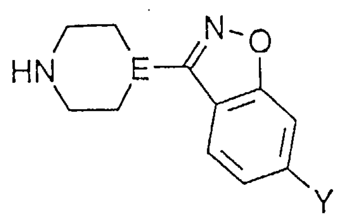

(II)

in der E und Y die in Anspruch 1 angegebenen Bedeutungen besitzen, entweder
mit einer Verbindung der Formel **III**

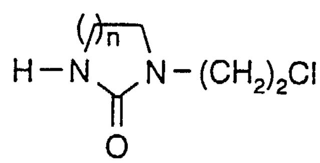

**(III)**

in der n die oben angegebene Bedeutung besitzt,
in Gegenwart eines Alkalimetallcarbonats in einem polaren Lösungsmittel umsetzt zur Bildung einer Verbindung der Formel **IV**

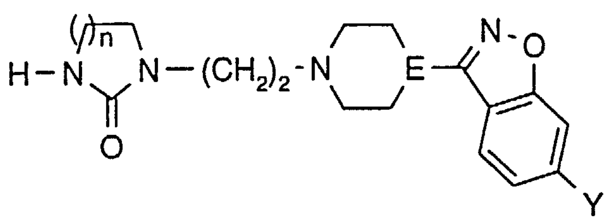

**(IV)**

in der n, E und Y die in Anspruch 1 angegebenen Bedeutungen besitzen, welche man anschließend mit einer Verbindung der Formel **V**

$$A\text{-}(CH_2)_m\text{-}X \qquad (V)$$

in der A und m die in Anspruch 1 angegebenen Bedeutungen besitzen und X ein Halogenatom darstellt,
in Gegenwart eines Alkalimetallhydrids in einem polaren Lösungsmittel umsetzt zur Bildung der Verbindungen der Formel **I**;

oder
mit einer Verbindung der Formel **VI**

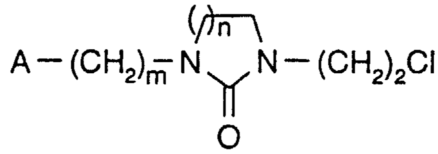

**(VI)**

in der A, m und n die in Anspruch 1 angegebenen Bedeutungen besitzen, in Gegenwart eines Alkalimetallcarbonats in einem polaren Lösungsmittel umsetzt zur Bildung der Verbindungen der Formel **I**.

**6.** Pharmazeutische Zubereitungen zur Behandlung von psychotischen Störungen, von anxio-depressiven Störungen und der Aggressivität enthaltend als Wirkstoff eine Verbindung nach einem der Ansprüche 1 bis 4 zusammen mit einem oder mehreren pharmazeutisch geeigneten Trägermaterialien.

## Claims

**1.** 1,2-Benzisoxazole compounds of formula **I**

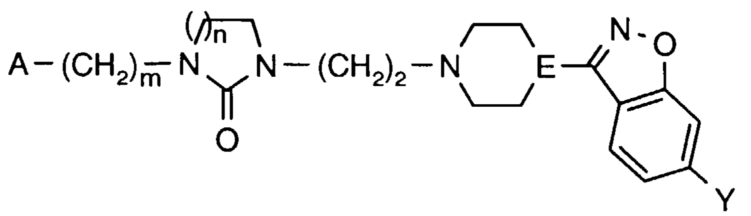

**(I)**

wherein:

A is linear or branched alkyl having from 1 to 10 carbon atoms or phenyl optionally substituted by one or more halogen, hydroxy or alkoxy,

m represents zero or 1,

n represents 1 or 2,

E represents N or CH, and

Y represents hydrogen, halogen or ($C_1$-$C_5$)alkoxy ;

and their optical isomers when they exist and addition salts thereof with a pharmaceutically acceptable acids.

**2.** A compound of claim 1 that is 1-{2-[4-(6-fluoro-1,2-benzisoxazol-3-yl)piperazin-1-yl]ethyl}-3-isopropylimidazolidin-2-one.

**3.** A compound of claim 1 that is 1-{2-[4-(6-fluoro-1,2-benzisoxazol-3-yl)piperazin-1-yl]ethyl}-3-ethylimidazolidin-2-one and the hydrochloride thereof.

**4.** A compound of claim 1 that is 1-{2-[4-(6-fluoro-1,2-benzisoxazol-3-yl)piperid-1-yl]ethyl}-3-phenylimidazolidin-2-one.

**5.** A process for the preparation of the compounds of claim 1, characterised in that:
a compound of formula **II**

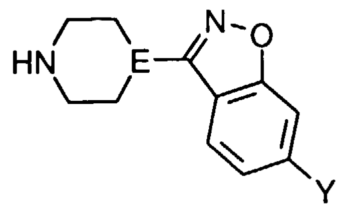

**(II)**

wherein E and Y are as defined in claim 1,
is reacted
<u>either</u>
with a compound of formula **III**

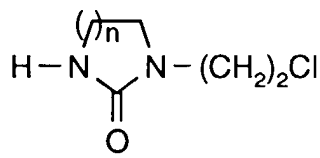

**(III)**

wherein n is as defined hereinbefore,
in the presence of an alkali metal carbonate in a polar solvent, to obtain a compound of formula **IV**

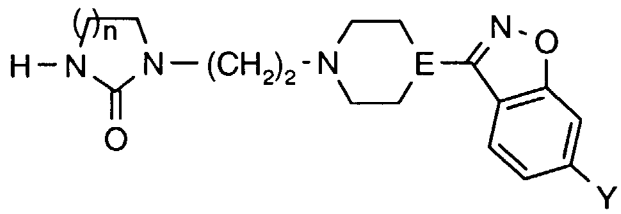

**(IV)**

wherein n, E and Y are as defined in claim 1,
which is then reacted with a compound of formula **V**

$$A-(CH_2)_m-X \qquad \textbf{(V)}$$

wherein A and m are as defined in claim 1 and X is a halogen atom,
in the presence of an alkali metal hydride, in a polar solvent, to obtain the compounds of formula I ;
<u>or</u>
with a compound of formula **VI**

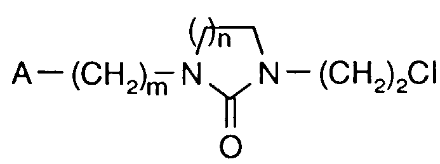

**(VI)**

wherein A, m and n are as defined in claim 1, in the presence of an alkali metal carbonate, in a polar solvent, to yield the compounds of formula **I**.

**6.** Pharmaceutical compositions that can be used in the treatment of psychotic disorders, anxio-depressive disorders and aggressiveness, comprising as active ingredient a compound according to any one of claims 1 to 4 with one

or more appropriate pharmaceutical excipients.